# EUROPEAN PATENT APPLICATION

(11) **EP 1 762 969 A2**
(43) Date of publication of application: **14.03.2007**
(21) Application number: 06014836.8
(22) Date of filing: 17.07.2006
(51) Int. Cl.: G06Q 10/00

(54) **Method and device for the automated dispensing of packaged medicines on prescription as well as a label to be used therewith**

(30) Priority: 15.07.2005 NL 1029537
(71) Applicant: Holding F.M. van der Vaart B.V., 5066 EK Moergentel (NL)
(72) Inventor: Van der Vaart, Franciscus Marie, 5066 EK Moergestel (NL)
(74) Representative: Dohmen, Johannes Maria Gerardus

(57) **Abstract**

A method and a device for the automated dispensing of packaged medicines on prescription, comprising the steps of: a) inputting (8) prescription data into an information processing system (7, 9, 10); b) producing (11; 12) a prescription provided with the input prescription data and a unique identification code associated therewith; c) reading (14; 12) the identification code of a prescription; d) retrieving the corresponding prescription data from the information processing system (7, 9, 10) on the basis of the identification code as read; e) automatically verifying (27) whether a packaged medicine corresponds to the retrieved prescription data; f) mechanically producing (28) one or more labels for an identified medicine according to the retrieved prescription data and mechanically applying (29) the same to the package of the medicine; g) registering (15) a dispensing code corresponding to a dispensed medicine with the prescription; and h) collecting (32) the medicines provided with a label for dispensing the same. The invention also relates to a label for use with the method and the device.

## Description

The invention relates to a method of and a device for an, as much as possible, automated dispensing of packaged medicines or drugs on prescription, in particular for use in pharmacies, drugstores and the like.

The dispensing of medicines or drugs nearly always takes place on the basis of a prescription. The most common form is a prescription on paper which, in addition to the patient's data, includes the name of the medicine, the instructions for the administration and, insofar as applicable, the dosage and the number of pills or powders of the medicine in question as well as any other information that may be relevant.

Besides the traditional paper prescription, more and more digital information processing systems are being used in practice, which enable prescribers of medicines, such as doctors, and dispensers of medicines or drugs , such as pharmacists or druggists, to exchange information directly in digital form. A paper prescription is no longer handed out in that case, but the prescription appears for example on a display screen connected to the information processing system at the location of the dispenser of the medicines.

At present practically all medicines are supplied in standard packages. It is inter alia the responsibility of the dispenser of the medicines not only to dispense the correct medicine in the correct amount, but also to inform the user or patient correctly of the information on the prescription regarding the instructions for the administration of the medication, i.e. the use of the medicine or drug. In practice generally one or more labels are applied to the packaged medicine, on which label(s) the instructions for the administration of the medication and any further information that may be relevant for the user are printed. In addition to that, separate patient information leaflets are often provided, which contain additional information regarding the use of the medicine and, for example, possible side effects thereof.

Although automated dispensing systems for collecting medicines from a stock or a warehouse, with the medicines being deposited in a container, are known, the labelling of packaged medicines is still substantially entirely a manual process in practice.

A problem that occurs when the labelling of packaged medicines is carried out by a machine is inter alia the fact that the dimensions of a label to be applied must be matched to those of the smallest package. Furthermore, there is always a risk that information present on the package, such as an identification code or other information that is relevant to a patient or user, will be hidden from view by the label. In such a case it will be necessary to remove the label in order to be able to read the respective information, in which case there is a great risk of damage not only to the label itself, but also to the text covered by the label, which may render it entirely illegible.

The manual labelling of medicines on the basis of a prescription is an error-prone process, in which errors may occur inter alia because instructions for the administration of the medication are incorrectly copied on a label and because labels and packages of a respective prescription are mixed up, which involves the risk that a user will incorrectly use a respective medicine, for example.

Furthermore, prescriptions and medicines may be mixed up, which may result in the wrong medicines, incorrect dosages, incorrect numbers etc. being provided to the wrong patients.

It will be appreciated that the dispensing of wrong medicines or the provision of incorrect instructions for the administration of medicines and the like may lead to serious medical complications or, for example, to a desired healing effect not being obtained.

In addition to these problems, manually dispensing medicines on prescription is a rather time-consuming activity involving relatively many operations, with a double check to be carried out by different persons independently of each other being indispensable in order to minimise the risk of errors and mistakes as much as possible.

Consequently it is an object of the present invention to provide a method and a device by means of which the dispensing of packaged medicines on prescription can take place in an automated manner, such that the number of operations to be carried out by persons is reduced as much as possible, in order to prevent errors as described in the foregoing as much as possible and in order to speed up the medicine dispensing process significantly in comparison with the above-described manual method, which is still being used on a large scale in practice.

In order to accomplish that object, the invention, in a first aspect thereof, provides a method for the automated dispensing of packaged medicines or drugs on prescription, comprising the steps of:
a) inputting prescription data into an information processing system;
b) producing a prescription provided with the input prescription data and a unique identification code associated therewith;
c) reading the identification code of a prescription;
d) retrieving the corresponding prescription data from the information processing system on the basis of the identification code as read;
e) automatically verifying whether a packaged medicine corresponds to the retrieved prescription data;
f) machined producing one or more labels for an identified medicine according to the retrieved prescription data and mechanically applying the same to the package of the medicine;
g) registering a dispensing code corresponding to a dispensed medicine with the prescription; and
h) collecting the medicines provided with a label for dispensing the same.

The invention is based on the perception that also in an automated process for the dispensing of prescribed medicines or drugs a uniquely identifiable prescription is indispensable to the dispenser of a medicine in order to be able to check in an adequate matter whether the correct medicines have been dispensed in the correct dosage and with the correct instructions for the use or administration thereof.

The mixing up of prescriptions is prevented when using the method according to the invention in that each prescription is provided with a unique identification code, which is associated one on one with the input prescription data in the information processing system. Preferably, this identification code is generated by the information processing system. It is also possible, however, to derive or copy the identification code from the prescription data itself, for example from the prescriber's data, such as a serial number, from the patient's or user's data, etc. The dispenser of the medicine himself may also input a unique identification code into the information processing system.

The prescription can be made available both electronically and on paper. In the case of an electronic prescription it is possible to store the prescription with the unique identification code on a removable data carrier, such as a memory card, a floppy disk, a so-called "memory stick" or other registration medium for the storage of data in digital form thereon. The identification code can be read from the data carrier in that case and prescription data retrieved from the information processing system on the basis of said identification code can subsequently be compared with the prescription on the data carrier for verification.

Instead of using a removable data carrier, the prescription in electronic form may also be transferred via a datalink, in which case the respective prescription data are retrieved from the information processing system on the basis of the transferred identification code and can be compared with the transferred prescription again for verification.

The method according to the invention furthermore advantageously comprises the producing of a prescription in paper form with a unique, machine-readable identification code associated therewith so as to enable machine-reading of the identification code of a paper prescription. A paper prescription enables a dispenser of medicines, such as a pharmacist or the like, to compare the medicines to be dispensed eventually with the prescription data without the use of further (electronic) means being required. Machine-readable codes for use with a paper prescription are known per se in practice, think for example of the generally known bar code.

By ensuring in accordance with an embodiment of the method according to the invention that the medicines that have been automatically collected on the basis of the input prescription data will only be released for dispensation when combined with the prescription in electronic or paper form, an important source of errors, viz. the mixing up of prescriptions and medicines, can be eliminated, for example.

An effective verification whether the correct medicines are in fact being dispensed is furthermore achieved in that a dispensing code corresponding to the code of the medicines collected for dispensation to a patient or user is registered with the electronic or paper prescription. Said dispensing code can be used for directly verifying whether the medicines according to the prescription data and the dispensed medicines correspond. This will not be the case when a prescription has been mixed up, for example.

Advantageously, the dispensing code is a code that is easy to read for a person, and in an embodiment of the invention a duplicate of the input prescription data is registered with the prescription. In the case of a prescription in paper form, for example, the dispensing code can be directly printed thereon or, for example, be provided thereon in the form of one or more labels containing information about the medicine, such as the name, the instructions for the administration thereof and other relevant information. Think in this respect of the labels that must also be applied to the packages of the medicines to be dispensed.

An important source of errors that occur when medicines are manually dispensed, viz. the incorrect copying of the instructions for the administration of the medication on the label and also the mixing up of labels is eliminated when using the method according to the invention, in that the labels are produced on the basis of the prescription data input into the information processing system and are applied in a fully automated manner to the respective medicines, which are in turn automatically identified and verified on the basis of the input prescription data.

In the most optimum embodiment of the invention, the medicines are directly collected and made available for labelling by an automatic medicine dispenser that is automatically controlled on the basis of the input prescription data.

In another embodiment, the invention provides a method wherein the medicines are collected on the basis of the prescription data and a respective medicine is subsequently machine-identified on the basis of a machine-readable article code that has been applied to the package thereof. The prescription data in question are then automatically examined to check whether the medicine in question is included therein, and if that is the case, the medicine in question is labelled, with the labels being produced in an automated process in accordance with the prescription data of the identified medicine. If the identified medicine is not included in the retrieved prescription data, no further processing thereof will take place, as the medicine apparently does not form part of the respective prescription. The medicines that have been provided with a label can subsequently be collected in a container.

The advantage of this embodiment is that the medicines can be collected both manually and by means of any kind of automatic medicine dispenser for further processing thereof in accordance with the prescription data. This renders the method according to the invention very flexible and versatile in use in practice.

For further verification whether the correct medicines are being dispensed, not only as regards the composition thereof but also as regards their origin, the method according to the invention comprises the further step of carrying out an authenticity check on the basis of a machine-readable authenticity code present on a packaged medicine in addition to the identification code for identifying the medicine and the composition thereof.

The authenticity code makes it possible to check the origin of a medicine, i.e. whether it is a legally, authentically produced medicine or an imitation product of unknown origin.

With a view to carrying out said authenticity check, the invention comprises the step of consulting, via a data communication network such as the Internet, a respective database comprising the relevant data concerning the composition and the origin of a respective medicine.

If the authenticity check and/or the identification of the composition of a medicine should fail, the process of dispensing the respective medicine will be discontinued.

Within the context of the present invention, the term prescription data is understood to mean all data relevant to the dispensing of the medicines, among which not only the data that directly relate to the medicines to be dispensed but also data relating to the prescriber, the patient or user and, if necessary, data relating to the dispenser of the medicines.

These data are preferably registered in an existing information processing system for medicines. Within the framework of the invention it is also possible, of course, to use an information processing system designed specifically for this purpose.

As already mentioned in the introduction, a prescriber may directly provide a patient or user with a prescription in paper form, on which the relevant data are written or printed. The dispenser of the medicine must input the prescription data into the information processing system in that case. A code that is already present on the paper prescription or an identification code generated by the information processing system may be used as the identification code of the prescription in that case, which code is subsequently compared with the prescription data in the information processing system.

In the former case, the step of producing a prescription in paper form in accordance with the method according to the invention need not be carried out again, because this has already been done by the prescriber. However, it is possible and - with a view to realising a uniform system structure and processing - advisable to produce a new or substitute paper prescription yet on the basis of the input data, which prescription is subsequently used for further carrying out the method according to the invention.

When the information processing system generates an identification code or, generally, when the identification code is not present on the existing paper prescription, the step of producing a prescription in paper form according to the invention comprises the registration of the identification code on the existing paper prescription. In this case, too, a completely new or substitute paper prescription may be produced, of course.

In the situation in which prescription data are presented to the dispenser of the medicine in fully digital form it is at all times also possible to produce a paper prescription containing all the relevant prescription data and the identification code.

Preferably, the prescription data and/or the identification code are printed on a paper carrier, using printer means that are known per se in practice.

In addition to unequivocally linking the prescription and the input prescription data, the identification code also makes it possible to unlink the inputting of the prescription data and the dispensing of the medicines from each other both physically and in time. For example, the prescription data can be input at an earlier point in time and, if desired, at a first location, and the prescription and the respective medicines can be produced and supplied at a later point in time and, if desired, at a second location. It will be understood that the method according to the invention, in addition to its higher degree of reliability, thus enables a more optimum and flexible operation in comparison with the current manual method of dispensing medicines on prescription.

In practice a number of exceptional situations may occur, for example a situation in which certain medicines are not in stock. These medicines must be delivered at a later date in that case. According to a further embodiment of the invention, a subsequent delivery of non-dispensed medicines is initiated by linking the subsequent delivery to the identification code in the information processing system. Preferably, a subsequent delivery note is produced, which is provided together with the medicines and a prescription in paper form.

If a respective label lacks sufficient space for providing the required information thereon, or if extra or additional information is to be provided to a patient or user, the method according to the invention provides the step of producing one or more further labels or information sheets or relevant documents, such as patient information leaflets, in accordance with the retrieved prescription data, for adding same to an identified medicine.

Furthermore, situations may occur in which specific medicines are not delivered in a standard package, for example, or in which a standard package must be reduced or split up. According to yet another embodiment of the invention, medicines may to that end be manually added to the container, for which medicines a further label and/or information sheet is produced in that case.

With the method according to the invention, the information on a label is preferably printed in different colours or colour fields, for example depending on the type of information that is provided, such as a warning text.

The invention furthermore relates to the provision of a receipt to be issued together with the medicines to be dispensed.

In a preferred embodiment of the invention, the dispensation of the medicines is directly recorded in the information processing system for the purpose of stock management and the like.

According to a second aspect of the invention, a device is provided for the automated dispensation of medicines or drugs on prescription, comprising:
- input means for inputting prescription data into an information processing system;
- means for producing a prescription provided with the input prescription data and a unique machine-readable identification code associated therewith;
- reading means for reading the identification code of a prescription;
- processing means for retrieving the corresponding prescription data from the information processing system on the basis of the identification code as read;
- identification means for automatically identifying and verifying whether a packaged medicine corresponds to the retrieved prescription data;
- label producing means for producing one or more labels for an identified medicine according to the retrieved prescription data;
- applicator means for mechanically applying a label that has been produced to the package of a respective medicine;
- registration means for registering a dispensing code corresponding to a dispensed medicine with the prescription; and
- collecting means for collecting the medicines provided with a label for dispensing the same.

The means for producing a prescription may be arranged for making the prescription available electronically and/or on paper.

In the electronic variant, the means for producing a prescription are arranged for storing the prescription on a removable data carrier, with the processing means being arranged for reading the identification code from the data carrier and verifying whether the retrieved prescription data and the prescription on the data carrier correspond.

In a further variant of the device according to the invention, rather than using separate data carriers, the means for producing a prescription are arranged for transferring the prescription via a data communication link, with the processing means being arranged for reading the identification code of the transferred prescription by electronic means and for verifying whether the retrieved prescription data and the transfer prescription data correspond.

In an embodiment of the device according to the invention, the registration means are arranged for electronically registering the dispensing code with the prescription in the case of a fully electronic or virtual prescription.

In the embodiment that uses a prescription in paper form, the means for producing a prescription are according to the invention arranged for producing a paper prescription with a unique, machine-readable identification code associated therewith, with the reading means being arranged for machine-reading the identification code of a paper prescription.

In a preferred embodiment of the device according to the invention, the identification means, the label producing means and the applicator means are arranged in series adjacent to a conveyor belt, with an inlet side located on the side of the identification means and an outlet side located on the side of the applicator means, wherein the collecting means are disposed on the outlet side and wherein the conveyor belt is arranged for conveying packaged medicines from the inlet side to the outlet side.

The conveyor belt is so dimensioned that it is capable of conveying most common packaged medicines. Think in this connection of medicines packaged in cartons, jars, strips and the like.

Electric driving means which are known per se are provided for driving and controlling the conveyor belt, which driving means are connected to control means for controlling the movement of the conveyor belt. The conveyor belt is preferably arranged for conveying medicines in two directions. The reason for this is that when the identification means identify a medicine that does not correspond with a prescription currently being processed, the medicine that has been placed on the conveyor belt is not moved in the direction of the collecting means but in the opposite direction, so that further processing of the respective packaged medicine will not take place.

In a preferred embodiment of the device according to the invention, sensor means are disposed on the inlet side of the conveyor belt for detecting the presence of a packaged medicine that has been placed on the conveyor belt and subsequently activating the conveyor belt for conveying the medicine either towards the outlet side or away from the outlet side, depending on whether it forms part of a respective prescription or not. All this as extensively described in the foregoing.

The identification means for identifying a packaged medicine placed on the conveyor belt are connected to the control means for the processing of packaged medicines, for example by controlling the direction of movement of the conveyor belt or by activating stop means and the like. Suitable identification means are arranged for the machine-reading of article codes on the packaged medicine, such as a bar code reader for reading article codes in the form of bars that are known per se in practice. Other machine-readable article codes may also be used, of course, and the identification means may be suitable for reading more than one type of article code, such as the aforementioned authenticity code, and consulting a respective database upon doing so.

In a preferred embodiment of the invention, the registration means for registering a dispensing code corresponding to a dispensed medicine on the paper prescription are disposed in such a manner with respect to the collecting means, such as a container, that after the dispensing code has been registered the paper prescription is added to the medicines collected in the collecting means and provided with a label to which the respective prescription relates. It is noted that the registration means may also produce a new paper prescription, in particular in the case of digital processing of the prescription and the prescription data.

That is, in accordance with the inventive concept the labelled medicines and the prescription are collected as a whole for being dispensed.

To prevent medicines being removed from the collecting means before the dispensing code has been placed on the paper prescription and been added to the collecting means, releasing means are according to the invention provided for releasing the collecting means, such as a container, in such a manner that the container cannot be removed until the packaged medicine and the paper prescription have been collected therein. A next prescription cannot be processed before the container or another collecting bin has been placed in the device for collecting a new paper prescription and the associated medicines.

Reading means are provided for reading a paper prescription, i.e. the unique identification code thereof, on the basis of which the prescription data are retrieved from the information processing system, which reading means are suitable for machine-reading the identification code, which in itself may be a bar code again or any other suitable code. In the case of a bar code, the reading means are suitable for reading bar codes, as known per se in practice. In this case, too, the reading means may be arranged for reading various machine-readable identification codes.

In a preferred embodiment of the device according to the invention, the reading means and the registration means are combined into one device, so that the prescription only needs to be input once for starting the medicine dispensing process, which is an aspect that functions to prevent further errors caused by the mixing up of prescriptions. Preferably, the reading means and the registration means are so arranged that the prescription is not released before all the available medicines have been collected in the collecting means.

To provide the packaged medicine with an associated label containing the relevant prescription data, as set forth in the foregoing, the label producing means are arranged for printing the respective data on an non-preprinted label. The printed label thus produced is subsequently applied to the package of a respective medicine by the applicator means.

In a preferred embodiment of the invention, a label is provided with affixing means or an adhesive medium, and the applicator means are arranged for affixing or adhering a respective label to the package of a medicine, for example by glueing or stapling or by means of a snap connection or the like.

Since the identification of a medicine, the production of the label and the application thereof to the packaged medicine take place in succession in a single operation, the risk of errors is minimal in this case as well, so that the correct label will be applied to the correct medicine.

In a preferred embodiment of the device according to the invention, further means are provided for producing further labels and/or information sheets to be added to the collecting means. Think in this connection of labels to be applied to medicines that cannot be provided with a label by the applicator means or medicines that are manually added to the collecting means, for example because they are not packaged as standard or because the standard package must be reduced or split up. Said further information sheets may consist of a patient information leaflet, a subsequent delivery note, a receipt or the like. Preferably, said further means, for example in the form of a label printer, a printer for e.g. A4 sheets and the like, are in turn so arranged that a further label and/or information sheet is (are) automatically added to the medicines in the collecting means upon being delivered.

In the most optimum embodiment of the device according to the invention, the device is directly connected to an automatic medicine dispenser, which automatically retrieves the medicines associated with a respective prescription from a stock and supplies them to the label producing means and the applicator means for labelling, after which the medicines are collected in the collecting means.

In an embodiment of the device according to the invention, which is especially suitable for manual identification of medicines being supplied, which may have been dispensed by an automatic medicine dispenser, for example, the reading means, the processing means, the identification means, the label producing means, the applicator means, the registration means, the collecting means, the sensor means and, if applicable, the conveyor belt and the further means for producing labels and/or information sheets are combined into a single, integrated device.

This integrated, combined device preferably comprises connecting means for connection to an information processing system, but it may also comprise its own information processing system for processing prescription data in accordance with the method of the invention, as discussed in the foregoing.

According to a third aspect of the invention, a label is furthermore provided for use with a method and/or a device according to the invention, which label is provided with an adhesive along a circumferential edge thereof for the purpose of firmly adhering the label to the package of a medicine.

An advantage of such a label is that it can be affixed to the package at a location thereon such that a patient or user will still be able to read important information present on said package. Another advantage of such a label is the fact that it can be printed or provided with the information on two sides, so that more information can be placed on a label, which may benefit the patient or user. Furthermore, the dimensions of the label may be larger than those of the package to which it is to be affixed. Since the label according to the invention hangs from the package like a kind of flag, as it were, the attention value of the label according to the invention is much higher than that of a usual label that is adhered to the package in its entirety.

In a preferred embodiment of the label according to the invention, the circumferential edge provided with an adhesive and the adhesive itself are transparent. In this way no information on the package of the medicine is hidden from view as a result of the label being placed thereon, because the location on the package to which the label is adhered remains legible for the patient or user.

The adhesive may be any adhesive suitable for firmly adhering the label, such as a paper label, to a housing of cardboard or plastic material, for example, in which a medicine is packaged. Transparent adhesive is known per se in practice and need not be explained in more detail to those skilled in the art.

In order to further elucidate the information present on the label to a patient or user, the label may comprise fields in different colours, on which the respective prescription data and additional data, such as warnings and the like, can be printed. Printing on the label may be in colour as well, of course.

In a preferred embodiment of the label according to the invention, the end of the label that is not provided with an adhesive is provided with a further adhesive medium having a low adhesive power so as to make it possible to adhere this end to a package more than once.

In this way the label can be folded round the package and be adhered thereto with the free end thereof. The use of an adhesive medium having a low adhesive power, such as a weak adhesive, makes it possible to detach the end of the label from the package or the label itself from the package and attach it thereto again without causing any damage thereto.

The invention also relates to a roll comprising a number of labels as described above.

The invention will now be explained in more detail on the basis of an embodiment of a device according to the invention and an implementation of the method according to the invention for use with such a device. It is noted that the invention is not limited to the embodiment of the device and the methods as discussed and shown herein, of course, but that several variants thereof can be realised without departing from the inventive concept.

Figure 1 is a schematic view of an embodiment of the device according to the invention.

Figure 2 is a schematic front view of a device according to the invention similar to that which is shown in figure 1.

Figure 3 is a schematic side view of the device according to the invention as shown in figure 2.

Figure 4 is a schematic view on a larger scale of a packaged medicine provided with a label according to the invention during the processing thereof in the device that is shown in figure 2.

Figure 5 is a schematic view on a larger scale of an embodiment of applicator means according to the invention for use in the device that is shown in figure 2.

Figures 6 and 7 schematically show an embodiment of the label according to the invention applied to the package of a medicine in various views.

Figures 8 and 9 are schematic side views of a preferred embodiment of a label according to the invention applied to the package of a medicine.

Figure 10 is a schematic flow diagram showing the main steps of the method according to the invention.

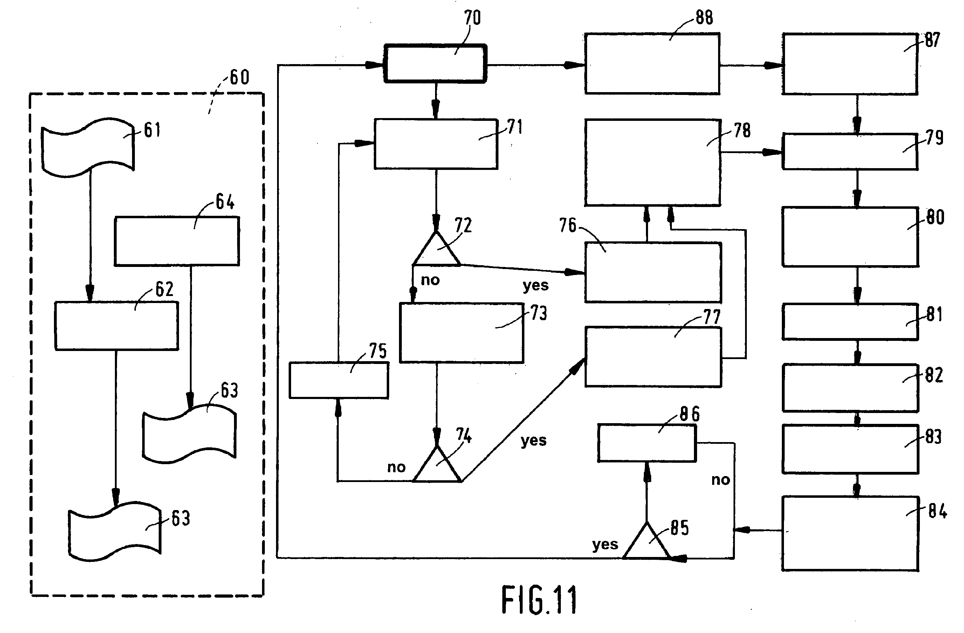

Figure 11 is a schematic view of display screen information for a user in accordance with the method according to the invention.

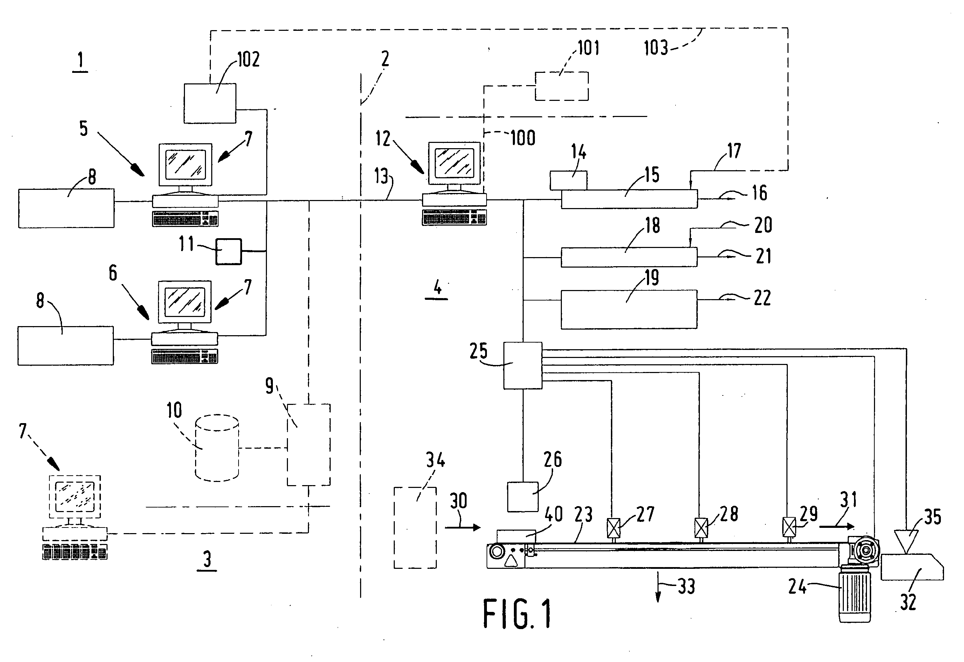

The device according to the invention that is schematically shown in figure 1 is indicated as a whole with 1. As already discussed in the introduction, the device according to the invention can be used at physically separate locations, which is schematically indicated by means of the vertical dashed line 2. Seen in the plane of the drawing, a prescription input part 3 is present on the left-hand side of the dashed line 2 and a medicine processing part 4 for the eventual dispensation of medicines in accordance with the prescription data input in the prescription input part 3 is present on the right-hand side of the dashed line 2. It will be understood that the parts 3 and 4 may also be combined into a single integrated device.

The prescription input part 3 comprises one or more input means 5, 6 in the form of, for example, a personal computer or desktop computer 7 that is known per se in practice, which is provided with suitable software. Reference numeral 8 indicates input means for inputting prescription data. The input means 8 may have any form that is suitable for inputting prescription data, among which a keyboard, means for the inputting of prescription data by speech or, for example, means for the machine-reading of printed text and the like.

An information processing system suitable for the processing of the prescription data may be installed on one computer or on several computers 7 or, for example, on a server 9 disposed at a central location with a database 10 connected thereto, if desired. The server 9 may be operatively linked to the personal computers 7, so that the server 9 and the database 10 may in fact be disposed at any location that may be desired.

A Personal Computer 7 of a prescriber, such as a family doctor or the like, may be connected to the server 9 via a suitable telecommunication link. This is indicated in broken lines in figure 1.

An information system for the registration and dispensation of medicines or drugs that is known per se or an information processing system especially developed for that purpose may be used for the processing of the prescription data in accordance with the invention.

The prescription data input part 3 furthermore comprises means 11 for producing, if so desired, a prescription in paper form provided with the input prescription data and a unique, machine-readable identification code associated therewith, such as a bar code, in accordance with the method of the invention. The means 11 may comprise a printer that is known per se, for example for printing data on a paper carrier in A6 format or another desired format.

Instead of printing an identification code directly on a paper prescription, the identification code may also be applied to the prescription by means of a label, for example, in which case the means 11 will comprise a label printer.

According to the invention, the input means 8 may be arranged for electronically registering a prescription with the unique identification code associated therewith on a data carrier, such as a floppy disk, a memory card, a "memory stick" or other registration means for the storage of digital data.

Furthermore, the computers 7 and/or the means 8 may be arranged for transferring the prescription with the associated identification code to the medicine processing part 4 via suitable communication means 102 over a data communication link 103, which may be a wire communication link or a wireless communication link.

The medicine processing part 4 comprises processing means 12 for retrieving input prescription data, which processing means, in one embodiment thereof, may consist of a suitably programmed personal computer or desktop computer that is known per se in practice, which is operatively linked to the prescription data input part 3, in particular the personal computers 7 and/or the server 9, via a telecommunication link. The processing means 12 may also be arranged for directly inputting prescription data, if desired, via input means 8 as disclosed in the foregoing.

The medicine processing part 4 further comprises reading means 14 connected to the processing means 12 for the machine-reading of the identification code of a paper prescription and registration means 15 for registering a dispensing code corresponding to a dispensed medicine on the paper prescription. In the case of an identification code in the form of a bar code that is known per se, the reading means 14 will comprise a bar code reader that is known per se. Other suitable machine-readable identification codes may be used as well, of course, in which connection it will be understood that the reading means 14 will in that case also be suitable for machine-reading the respective code or codes.

In an embodiment of the invention, the registration means 15 comprise a printer for printing the dispensing codes directly on the paper prescription, for example on the non-printed back thereof. The registration means 15 are so arranged that the paper prescription is automatically discharged from the registration means 15 after the registration of the dispensing code or codes, as is schematically indicated by the arrow 16 in figure 1. The arrow 17 indicates an inlet side of the registration means 15 for inserting the paper prescription therein. The reading means 14 and the registration means 15 are advantageously configured as a single, integrated unit, so that a paper prescription only needs to be inserted once for reading the identification code and subsequent registering the dispensing code or codes on the paper prescription.

In the case of an electronically available prescription, the reading means 14 according to an embodiment of the invention are suitable for reading the prescription and the associated identification code from a data carrier of the kind referred to in the foregoing, or directly electronically via the data communication link 103. Reading means that are suitable for this purpose are known per se in practice and need not be explained in more detail to those skilled in the art.

As already indicated in the introduction, it may be necessary to print additional information intended for being added to medicines or drugs to be dispensed, to which end further means 18 may selectively be provided, for example in the form of an A4 printer having an inlet side 20 for introducing pre-printed paper, for example, and an outlet side 21 for automatically delivering printed paper, such as a receipt, a subsequent delivery note, in the case that medicines must be delivered at a later date, and the like. The further means 19 may comprise a label printer that is known per se, comprising an inlet side 22 for printed labels, for example labels intended for application to medicines that are not packaged as standard.

The medicine dispensing part 4 is concentrated around a conveyor belt 23, which conveyor belt is driven by an electric motor 24, which motor is controlled by electronic control means 25. Like the reading means 14, the processing means 15 and the further means 18, 19, the control means 25 may be operatively connected to the processing means 12 for mutually exchanging control information and prescription data or other control and information signals necessary for the operation of the device according to the invention.

Disposed adjacent to the conveyor belt 23 are, successively, sensor means 26, identification means 27, label producing means 28 and applicator means 29. The inlet side 30 of the conveyor belt 23 is located near the sensor means 26, and the outlet side 31 is located near the applicator means 29, with collecting means 32 in the form of a container being disposed at the outlet side 31 of the conveyor belt.

Medicines that correspond to retrieved prescription data are conveyed in the direction indicated by the arrow from the inlet side 30 to the outlet side 31 of the conveyor belt 23. Reference numeral 33 indicates that medicines that do not correspond to the retrieved prescription data are not conveyed to the outlet side 31 and are not further processed for being dispensed.

The embodiment of the device 1 according to the invention that is shown in figure 1 is suitable for manual placement of medicines to be dispensed on the conveyor belt 23 at the inlet side 30 thereof. In addition to that, the device may be coupled to or disposed near an automatic medicine dispenser that is known per se in practice, which automatically places the respective medicines on the conveyor belt 23. Such an automatic medicine dispenser is schematically indicated in broken lines at 43 in figure 1. An automatic medicine dispenser 34 that is suitable for this purpose is known by the trade name RoboPharma^{®}, for example. Such an automatic medicine dispenser 34 and the device 1 according to the invention may be combined into one unit, if desired.

The sensor means 26 are arranged for detecting the presence of a packaged medicine or drug that has been placed on the conveyor belt 23, they may for example comprise a photocell detector that is known per se.

The identification means 27 are arranged for machine-reading an article code or article codes on the package of a medicine that has been placed on the conveyor belt 23, they may for example comprise a bar code reader that is known per se. The identification means 27 may also be arranged for machine-reading other kinds of article codes, for example.

In an embodiment of the device according to the invention, the identification means 27 are in particular arranged for consulting a database 101, via the control means 25 and/or the processing means 12, by means of a data communication network 100, such as the Internet, which database comprises data regarding the origin or the authenticity of a respective medicine and, if necessary, the composition thereof. The database 101 may consist of several databases from several manufacturers of medicines. Such a verification makes it readily possible to ascertain whether a respective medicine originates from a respective medicine manufacturer or whether the medicine is an imitation product. The authenticity code is a unique, machine-readable code associated with a packaged medicine by means of which any medicine can be individually identified.

The label producing means 28 are arranged for producing a label to be affixed to the package of the medicine that has been placed on the conveyor belt 23, which label is provided with the information that is relevant for the use of the medicine, such as the instructions for the administration thereof and further data, such as the name of the patient or user, the name of the prescriber, generally a doctor, and if necessary the name of the dispenser of the medicine, for example the name of a pharmacist, a druggist or the like. In an embodiment of the device according to the invention, the label producing means consist of a label printer.

The applicator means 29 are arranged for applying a label produced by the label producing means 28 to the package of the medicine.

The sensor means 26, the identification means 27, the label producing means 28 and the applicator means 29 are operatively connected to the control means 25 for exchanging suitable control signals, prescription data and other control signals necessary for the operation of the device.

Reference numeral 35 indicates locking/releasing means that engage the container 32, which are likewise electrically operatively connected to the control means 25 for locking/releasing the container 32.

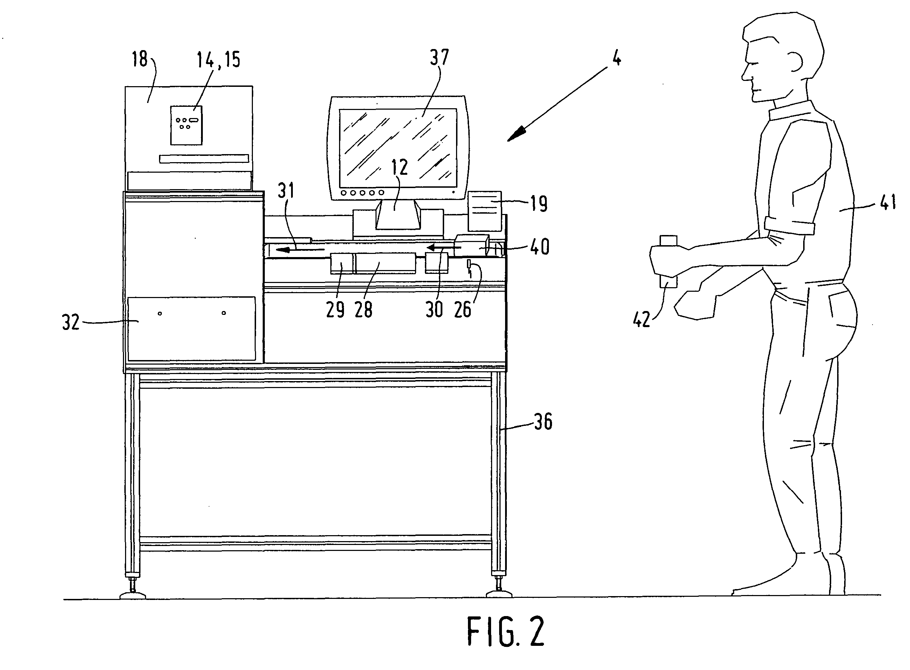

Figure 2 is a front view of an embodiment of the medicine processing part 4 of the device according to the invention.

Reference numeral 36 indicates a frame or table, on the left-hand side of which the reading means 14, the registration means 15, the further means 18, 19 and the collecting means or container 32 are disposed one above the other, seen in the plane of the drawing. The reading means 14 and the registration means 15 are again combined into one apparatus, as described in the foregoing. Also the further means 18, 19 for printing on A4 paper, for example, and for producing further labels are combined into one apparatus in this embodiment.

On the right-hand side of the table 36, the sensor means 26, the identification means 27, the label producing means 28 and the applicator means 29 are arranged one behind the other adjacent to the conveyor belt 23, seen from the inlet side 32 the outlet side 31 thereof. The container 32 connects to the outlet side 31 of the conveyor belt 23.

The processing means 12 are provided with a touchscreen 37, for example, for controlling the device by touching the screen. In the illustrated embodiment, the control means 25 are integrated in the processing means 12.

The electric driving means 24 for the conveyor belt 23 are not explicitly shown in figure 2.

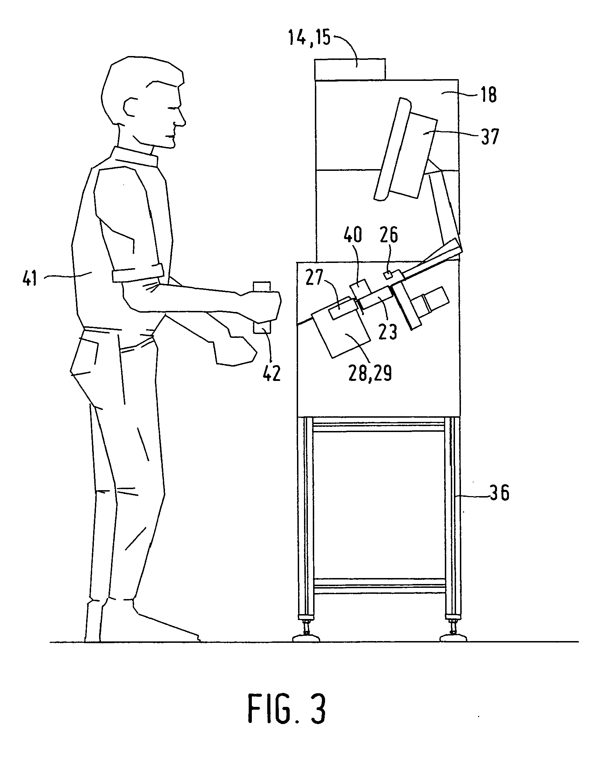

Figure 3 is a view of the right-hand side, seen in the plane of drawing, of the medicine processing part 4 according to the embodiment of figure 2.

Reference numeral 40 indicates a packaged medicine or drug present on the conveyor belt 23, which, in the illustrated embodiment of the medicine processing part 4 of the device according to the invention, may have been manually placed on the conveyor belt 23 by a person 31. Reference numeral 42 indicates a further packaged medicine, which may be placed on the conveyor belt 23 after the packaged medicine 40 has been processed, etc.

As already explained in the foregoing, the packaged medicines 40, 42, instead of being manually placed on the conveyor belt 23, may also be placed on the conveyor belt 23 by means of an automated medicine dispenser, which can be directly controlled on the basis of the prescription data for placing the respective packaged medicines on the conveyor belt 23. Since the identification of the medicines can be carried out by the automatic medicine dispenser, the identification means 27 need not be provided, if desired. In order to keep the degree of reliability of the dispensing process as high as possible, however, it is preferable to retain the identification means 27 for verifying additionally whether packaged products that have been placed on the conveyor belt 23 by the automatic medicine dispenser actually correspond to the prescription data and for carrying out the authenticity check as referred to in the foregoing.

Instead of directly being placed on the conveyor belt 23 by an automatic medicine dispenser, medicines may also be collected in a separate bin or container first, after which the respective packaged medicines 40, 42 are manually placed on the conveyor belt 23 by the person 41.

If an automatic medicine dispenser is used for delivering the packaged medicines directly to the conveyor belt 23, the medicine processing part 4 of the device 1 according to the invention may be combined into one unit (not shown) with such an automatic medicine dispenser.

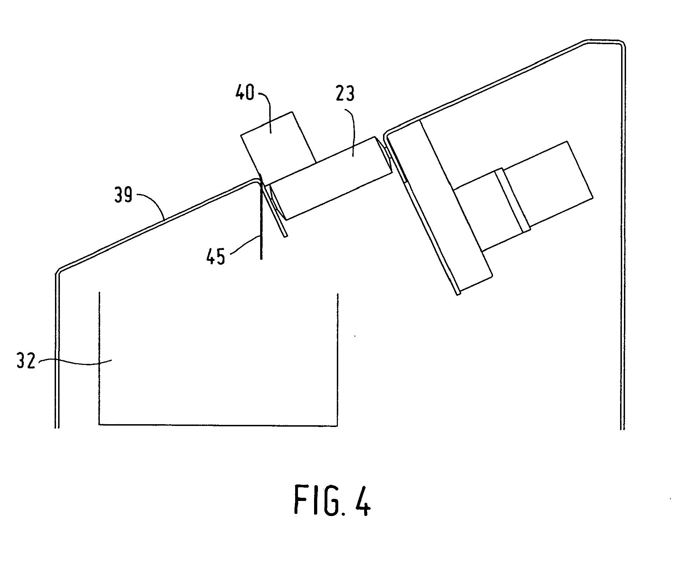

Figure 4 is a sectional view, on a larger scale than in figure 2, showing the conveyor belt 23 at the moment a packaged medicine 40 provided with a label 45 is being deposited into the bin 32 at the outlet side 31 of the conveyor belt 23. Reference numeral 39 indicates a product stop disposed on one side of the conveyor belt, against which a packaged medicine 40 on the conveyor belt 23 can strike.

Before a label 45 is affixed to the packaged medicine 40, it has been provided in the label producing means 28 with the relevant data for the respective medicine, such as the name of the medicine, instructions for the administration thereof, information concerning side effects or risks and the like, the name of the patient or user, the name of the prescriber, the name of the dispenser of the medicine, etc.

Unlike the usual labels that are adhered to a medicine package in their entirety, the label 45 according to the invention is only adhered to the package 40 of the medicine with one edge 51 thereof.

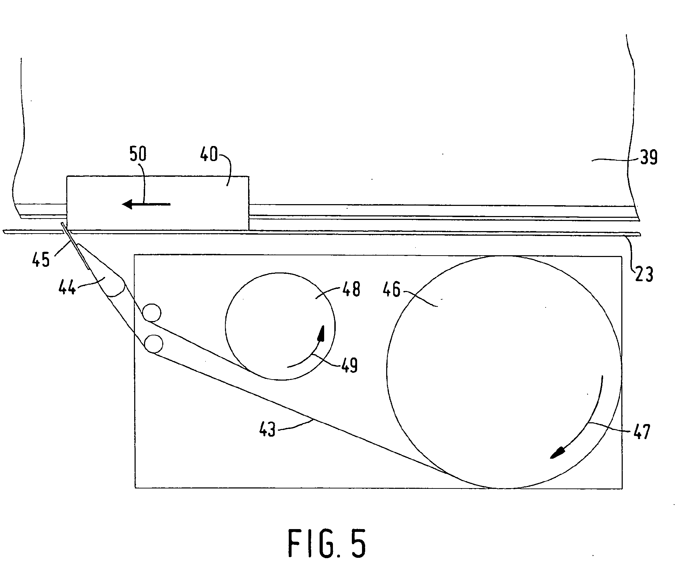

As is schematically shown in Figure 5, on a larger scale than in figure 2, the labels 45 to be affixed to a packaged medicine 40 are present on a carrier 43 on a supply roll 46 that rotates in the direction indicated by the arrow 47. The empty carrier 43 is collected on a collecting roll 48, which rotates in the direction indicated by the arrow 49. An applicator body 44 is provided for applying a label 45, which applicator body is positioned in such a manner that when the carrier 43 provided with the labels moves past the applicator body 24, the label 45 automatically comes off the carrier 43 for being applied to the packaged medicine 40 that is moving ahead in the direction indicated by the arrow 50. It will be understood that the conveying velocity of the carrier 43 must be tuned to the velocity of the conveyor belt 23 in this embodiment of the applicator means.

An alternative for the application of a label 45 to the package 40 of the medicine is the use of a so-called mechanical tamp, wherein the label 45 is removed from the carrier 43 and placed on the package 40 by making use of a vacuum.

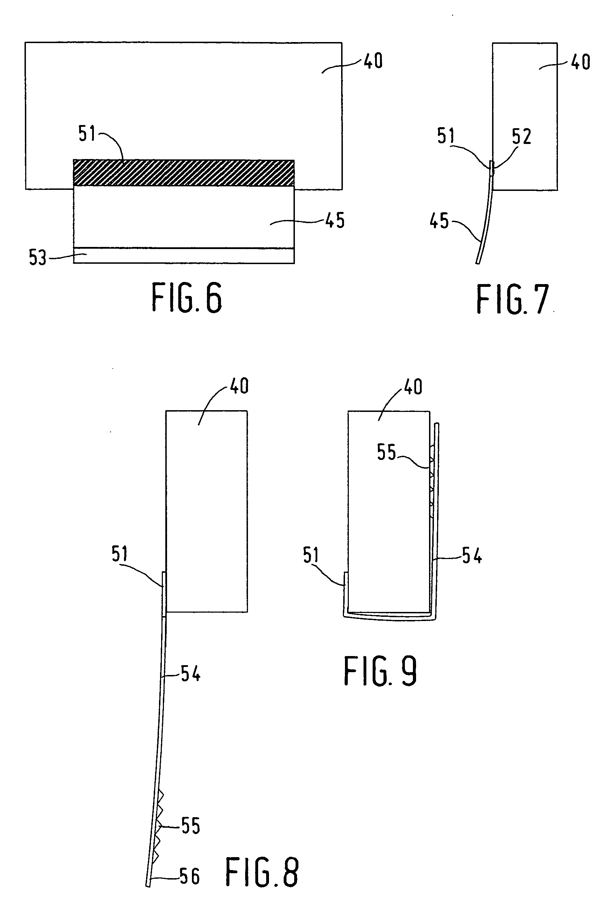

The placement of the label 45 according to the invention on a packaged medicine 40 is shown in more detail in figure 6 and figure 7.

Figure 6 is a front view of a packaged medicine 40 with a label 45 according to the invention affixed thereto. The label 45 is to that end provided with an adhesive medium 52 at a circumferential edge 51 thereof, as is shown in figure 7. Adhesive media suitable for the purpose of the invention for adhering the label 45 to a packaged medicine 40, such as a medicine packaged in a cardboard or plastic housing, are known per se in practice.

In contrast to a label that is glued to the package in its entirety, the label 45 according to the invention has this advantage that only a small part of the package 40 of the medicine is covered, i.e. can no longer be read.

In a preferred embodiment of the invention, a label 45 is provided wherein the adhesive edge 51 is transparent, as is the adhesive itself. Thus it remains possible to read the text on the package 40, without any loss of information that may be of relevant to a patient or user or with regard to the mechanical processing thereof. Furthermore, the label 45 according to the invention has this advantage that it can be printed on two sides, if necessary, because it will remain possible to read the label on two sides. In this way it is possible to print more information that may be relevant to a patient or user on a label of usual dimensions.

Figure 8 is a schematic side view of a label 54 according to a preferred embodiment of the invention, which, supplementary to the label 45, is provided with a weakly adhesive layer 55 near the end 56 of the label 54 remote from the circumferential edge 51. That is, an adhesive having an adhesive power such that a label 54 affixed by means of said adhesive layer can be detached from and be attached to a package 40 again several times without causing damage to the package or to the label 54. Adhesives suitable for this purpose are known per se in practice.

Figure 9 is a side view of the label 54, which is folded round the package 40 and which adheres to the package 40 via the adhesive layer 55. The adhesive power of the adhesive of the adhesive layer 55 is such that the label 54 thus folded round the package 40 is kept in place.

The label 45, 54 may be provided with one or more colour fields 53, for example at the longitudinal edge(s) thereof, for printing additional information thereon or information that merits extra attention.

It is possible, of course, to affix more than one label 45, 54 according to the invention to a package 40 whilst retaining the advantage that information that is present on the package can still be read.

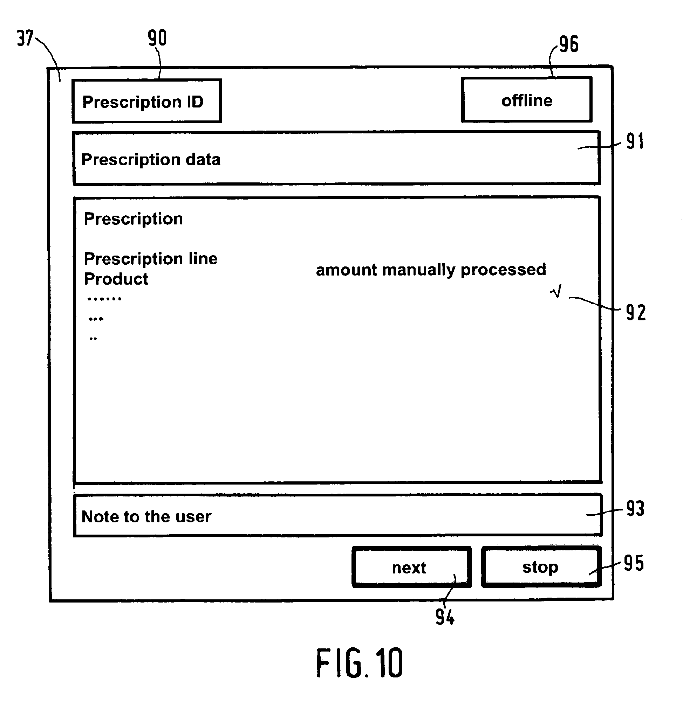

The operation of the device according to the invention will now be illustrated in more detail with reference to the flow diagram that is shown in figure 10 in combination with the embodiment of the device 1 that is schematically shown in figure 1, based on the use of a paper prescription.

Block 60 in figure 10 relates to the prescription input part of the device according to the invention.

A prescription may be issued directly on paper 61, i.e. in writing, by a prescriber. That is, the prescriber, such as a doctor, writes a paper prescription 61 containing the relevant data of the medicine and the instructions for the administration thereof, the name of the patient or user, the name of the prescriber and any other prescription data that may be relevant. Subsequently the user takes this paper prescription 61 to a dispenser of medicines, such as a chemist or a druggist, who inputs the prescription data into an information processing system (block 62).

After the respective prescription data have been input, the paper prescription 61 is provided with a unique identification code, for example in the form of a bar code, which is printed on the prescription, resulting in a paper prescription 63. Use may be made of the printing means 11 that are shown in figure 1 for printing the bar code on the paper a prescription 61. The input means 8 are provided for inputting the prescription data, as discussed in the foregoing with reference to figure 1. The information processing system may be installed on the computers 7, 12 and/or on the server 9.

When a prescriber sends a receipt to a dispenser of medicines in fully digital form 64, said inputting of the prescription data is no longer necessary, of course, and a paper prescription 63 provided with a unique identification code, such as a bar code, may be directly printed.

Instead of using a bar code as the unique identification code, any other identification code present on a paper prescription 61 may be used for this purpose, such as a serial number, for example supplemented with information relating to the prescriber or the patient. A condition in this regard is that the identification code must be machine-readable. The step of printing an identification code on the paper prescription is left out in that case. With a view to realising an unequivocal and reliable process, however, it is possible to print an entirely new paper prescription 63 for further processing in the device according to the invention.

What is important is that the paper prescription that is produced in block 60 be provided with the relevant prescription data and a unique identification code for the further processing of the prescription in the medicine processing part 4 of the device according to the invention.

The method according to the invention starts (block 70) with the insertion 17 of a paper prescription 63 produced in block 60 into the combined reading and registration means 14, 15.

After the identification code of the prescription, to be referred to below as the ID code, has been read (block 71), the processing means 12 verify (block 72) whether the ID code is known in the information processing system 12 of the invention.

If the ID code is not known therein (reply "no" in decision block 72), a further attempt will be made (block 73) to retrieve the prescription data from the personal computers 7 or the server 9 via the data communication link 13, as is shown in figure 1.

If an ID code of the respective prescription cannot be found (reply "no" in decision block 74), a message to that effect will be displayed on the display screen 37 of the medicine processing part 4 (block 75), so that an operator can interfere manually, if desired. Think in this connection of inputting a respective identification code manually, for example, or of inputting the prescription data entirely anew.

If it appears in decision block 72 that the ID code of the paper prescription is available in the processing means 12 (reply "yes" in decision block 72), the respective prescription data can be retrieved (block 76). If it appears in decision block 74 that the ID code of the paper prescription is available in the computers 7 or the server 9 (reply "yes" in decision block 74), the prescription data can be retrieved therefrom (block 77) and be input into the processing means 12.

The retrieved prescription data 76, 77 are displayed on the display screen 37, enabling the operator of the device to make a choice (block 78) between manual or automatic processing of the medicines. Generally, this choice will be automatic processing of the medicines, and this processing is started by placing the packaged medicine 40 on the conveyor belt 23. The sensor means 23 detect the presence of a product that has been placed on the conveyor belt and start an identification cycle (block 79).

The identification means 27 identify a respective packaged medicine on the conveyor belt 23 and verify, via the processing means 12, whether the medicine is known in the retrieved prescription data. If such is the case, the conveyor belt 23 is driven to convey the packaged medicine 40 to the label producing means 28, where a label 45 is produced, which label is subsequently applied to the packaged medicine 40 by the applicator means 29 (block 80).

If the identification means 27 identify the presence on the conveyor belt 23 of a medicine that does not correspond to the prescription data, the processing of the respective medicine is discontinued and the conveying direction of the conveyor belt is reversed by the control means 25, for example, or stop means (not shown) for stopping a package are activated.

After all the medicines of a respective prescription have been processed (block 81), this is registered, for example for the purpose of stock control, in the processing means 12 (block 82), and further prints, such as receipts, patient information leaflets or labels containing further information, are made if necessary, by the further means 18, 19. Furthermore, a subsequent delivery note may be made if it has been determined in block 81 that not all the medicines included in the prescription data can be dispensed.

After these further prints have been made (block 83), the dispensed medicines are provided with a dispensing code on the paper prescription 63 (block 84). In a preferred embodiment of the invention, the dispensing code consists of a text capable of being read by a person, which contains the data relating to the medicines that are mentioned on the paper prescription. Preferably, the dispensing codes are printed on the non-preprinted back of the paper prescription 63.

The medicine dispensing cycle is thus complete, and the prescription 63 thus provided with a dispensing code, the further prints and the labelled medicines are collected in the collecting bin 32.

Since the registration means 15 and the further printing means 18, 19 are disposed above the container 32 in the embodiment that is shown in figures 2 and 3, the documents in question can be directly collected in the container 32 after being released by the registration means 15 and the further means 18, 19. In this way the mixing up of a prescription and the associated medicines is prevented in an effective manner.

Once the receipt, the respective medicines and the further information, insofar as applicable, have been collected in the container 32, the releasing means 35 are driven to release the container 32 for the removal thereof and the dispensing of the medicines and information.

In decision block 85 it is verified whether the collecting bin 32 has been placed back in the device again, after which a new medicine dispensing cycle can be started (reply "yes" in decision block 85). As long as the container 32 or another collecting bin has not been placed back, a message to that effect is displayed on the display screen 37 (block 86), and a further processing cycle cannot be started (reply "no" in decision block 85).

Block 87 is provided for inputting prescription data in the case wherein the data are not available in the information processing system of the processing means 12 or the personal computers 7 or the server 9. In block 88 the operator can decide whether or not further information, such as further labels, receipts and the like, is to be produced.

Figure 9 is a view of the display screen 37 in an embodiment of the invention. The display screen may for example display the ID code 90 of the prescription, the prescription data 91 and the information 92 on a respective label. Messages regarding errors or other information relating to the processing of medicines are displayed in block 93, and the operator can start a new medicine dispensing cycle or terminate the current cycle by means of touch keys. A touch key 96 is provided for the case wherein the system does not have a communication link or for carrying out or starting actions manually.

Depending on the processing stage, for example, further information and different information may be displayed on the display screen 37, of course.

For fully electronically processing a prescription is will be appreciated that the steps described in the foregoing in relation to a prescription in paper form are basically substituted for corresponding or similar steps in relation to the prescription in electronic form.

Although the invention has been explained on the basis of a preferred embodiment in the foregoing, it will be appreciated by those skilled in the art that various modifications and/or additions are possible without departing from the inventive concept.

## Claims

1. A method for the automated dispensing of packaged medicines on prescription, comprising the steps of:
a) inputting prescription data into an information processing system;
b) producing a prescription provided with said input prescription data and a unique identification code associated therewith;
c) reading the identification code of a prescription;
d) retrieving the corresponding prescription data from the information processing system on the basis of said identification code as read;
e) automatically verifying whether a packaged medicine corresponds to said retrieved prescription data;
f) machined producing one or more labels for an identified medicine according to said retrieved prescription data and mechanically applying the same to the package of said medicine;
g) registering a dispensing code corresponding to a dispensed medicine with said prescription; and
h) collecting said medicines provided with a label for dispensing the same.

2. A method according to claim 1, wherein the prescription is made available electronically in step b).

3. A method according to claim 2, further comprising the step of storing the prescription on a removable data carrier for reading the identification code in step c) and verifying whether the retrieved prescription data and the prescription on the data carrier correspond.

4. A method according to claim 2, further comprising the step of transferring the prescription via a data communication link for the purpose of electronically reading the identification code of the transferred prescription in step c) and verifying whether the retrieved prescription data and the transferred prescription correspond in step d).

5. A method according to claim 2, 3 or 4, wherein the dispensing code is electronically registered with the prescription in step g).

6. A method according to claim 1, wherein a paper prescription with a unique, machine-readable identification code associated therewith is produced in step b) so as to enable machine reading of the identification code of a paper prescription in step c).

7. A method according to claim 6, wherein the dispensing code is registered on the paper prescription in step g).

8. A method according to claim 6 or 7, wherein the prescription data are input into the information processing system by a prescriber in step a) and wherein the paper prescription is produced in step b) by registering the input prescription data and the identification code associated therewith on a paper carrier.

9. A method according to claim 6, 7 or 8, wherein the prescription data are input into the information processing system on the basis of a supplied paper prescription by a prescriber in step a) and wherein the paper prescription is produced in step b) by registering an identification code associated with the input prescription data on the existing paper prescription, if necessary.

10. A method according to any of the claims 6-9, wherein the machine-readable identification code is in the form of a bar code.

11. A method according to any of the claims 6-10, wherein the prescription data and/or the identification code is (are) registered in step b) by printing the same on a paper carrier.

12. A method according to any of the preceding claims, wherein the medicines are identified by an automatic medicine dispenser to enable the subsequent execution of steps e) - h).

13. A method according to any of the claims 1-11, wherein the medicines are collected on the basis of the prescription data in step e) and a respective medicine is subsequently machine-identified on the basis of a machine-readable article code present on the package thereof, wherein said prescription data are automatically examined to check whether said medicine is included therein, wherein said medicine is subjected to step f) if such is the case and wherein the processing of said medicine is discontinued if such is not the case.

14. A method according to claim 13, wherein the medicines can be collected manually and/or by means of an automatic medicine dispenser.

15. A method according to claim 12, 13 or 14, wherein one or more further labels or information sheets or other relevant documents are produced in accordance with the retrieved prescription data in step f) for addition thereof to an identified medicine.

16. A method according to claim 15, wherein medicines can be manually added to a container, for which medicines a further label and/or an information sheet and/or other documents is (are) produced.

17. A method according to any of the preceding claims, wherein a subsequent delivery of non-dispensed medicines is initiated by associating the subsequent delivery to the identification code in the information processing system.

18. A method according to claim 17, wherein a subsequent delivery note is produced, which is provided together with the medicines in step h).

19. A method according to any of the preceding claims, wherein the dispensing code for the supplied medicines is registered with the prescription in the form of a duplicate of the respective prescription in step g).

20. A method according to any of the preceding claims, wherein the dispensing code for the supplied medicines is placed on a paper prescription in step g) in the form of a label produced for the supplied medicines.

21. A method according to any of the preceding claims in dependence on claim 12 or 13, wherein a container is released for dispensation of medicines once a prescription in electronic or paper form provided with an identification code and, insofar as applicable, further labels, information sheets, documents and/or a subsequent delivery note have been collected therein in addition to the labelled medicines.

22. A method according to any of the preceding claims, wherein a receipt to be dispensed with the collected medicines is produced in step h).

23. A method according to any of the preceding claims, wherein the dispensation of the medicines is step h) is recorded in the information processing system for the purpose of stock management.

24. A method according to any of the preceding claims, wherein the information on a label is printed in different colours or colour fields.

25. A method according to any of the preceding claims, wherein an authenticity check is carried out in step e) on the basis of an authenticity code present on a packaged medicine.

26. A method according to claim 25, wherein said authenticity check is carried out by consulting, via a data communication network such as the Internet, a respective database comprising data concerning the composition and the origin or authenticity of a respective medicine.

27. A device for the automated dispensing of medicines on prescription, comprising:
- input means for inputting prescription data into an information processing system;
- means for producing a prescription provided with said input prescription data and a unique machine-readable identification code associated therewith;
- reading means for reading the identification code of a prescription;
- processing means for retrieving the corresponding prescription data from the information processing system on the basis of said identification code as read;
- identification means for automatically identifying and verifying whether a packaged medicine corresponds to said retrieved prescription data;
- label producing means for producing one or more labels for an identified medicine in accordance with said retrieved prescription data;
- applicator means for mechanically applying a label that has been produced to the package of a respective medicine;
- registration means for registering a dispensing code corresponding to a dispensed medicine with said prescription; and
- collecting means for collecting said medicines provided with a label for dispensing the same.

28. A device according to claim 27, wherein the means for producing a prescription are arranged for making the prescription electronically available.

29. A device according to claim 28, wherein the means for producing a prescription are arranged for storing the prescription on a removable data carrier, with the processing means being arranged for reading the identification code from the data carrier and verifying whether the retrieved prescription data and the prescription on the data carrier correspond.

30. A device according to claim 28, wherein the means for producing a prescription are arranged for transferring the prescription via a data communication link, with the processing means being arranged for reading the identification code of the transferred prescription by electronic means and for verifying whether the retrieved prescription data and the transfer prescription data correspond.

31. A device according to claim 28, 29 or 30, wherein the registration means are arranged for electronically registering the dispensing code with the prescription.

32. A device according to claim 27, wherein the means for producing a prescription are arranged for producing a paper prescription with a unique, machine-readable identification code associated therewith, with the reading means being arranged for machine-reading the identification code of a paper prescription.

33. A device according to any of the claims 27-32, wherein the identification means, the label producing means and the applicator means are arranged in succession adjacent to a conveyor belt, with an inlet side located on the side of the identification means and an outlet side located on the side of the applicator means, wherein the collecting means are disposed on the outlet side and wherein the conveyor belt are arranged for conveying packaged medicines from the inlet side to the outlet side.

34. A device according to claim 33, wherein sensor means are disposed on the inlet side of the conveyor belt for detecting the presence of a packaged medicine that has been placed on the conveyor belt and subsequently activating the conveyor belt for conveying said medicine either towards the outlet side or away from the outlet side.

35. A device according to any of the claims 27-34, wherein the registration means are arranged for delivering a paper prescription to the collecting means.

36. A device according to any of the claims 27-35, wherein the collecting means are configured as a container, and releasing means for releasing the container.

37. A device according to any of the claims 27-36, wherein the reading means and the registration means are combined into one device.

38. A device according to any of the claims 27-37, wherein the identification means are arranged for identifying a packaged medicine on the basis of a machine-readable article code present on the package of said medicine.

39. A device according to claim 38, wherein the identification means are arranged for verifying the authenticity of a packaged medicine on the basis of a machine-readable authenticity code present on the package of said medicine by consulting a respective database via a data communication network, such as the Internet.

40. A device according to any of the claims 27-39, wherein the label producing means are arranged for printing relevant prescription data on an non-preprinted label.

41. A device according to any of the claims 27-40, wherein a label is provided with affixing means and the applicator means are arranged for affixing a label that has been produced to the package of a medicine.

42. A device according to any of the claims 27-41, comprising further means for producing further labels and/or information sheets to be added to the collecting means

43. A device according to any of the claims 27-42, wherein the reading means, the processing means, the identification means, the label producing means, the applicator means, the registration means, the collecting means, the sensor means and, if applicable, the conveyor belt and the further means for producing labels and/or information sheets are combined into a single, integrated device.

44. A device according to any of the claims 27-43, further comprising an automatic medicine dispenser for identifying packaged medicines.

45. A label for use with a method and/or a device according to any of the preceding claims, provided with an adhesive along a circumferential edge thereof for adhering said label to the package of a medicine.

46. A label according to claim 45, wherein the circumferential edge provided with the adhesive and the adhesive itself are transparent.

47. A label according to claim 46, wherein said adhesive is a transparent adhesive.

48. A label according to claim 45, 46 or 47, wherein the end of the label that is not provided with the adhesive is provided with a further adhesive medium having a low adhesive power so as to make it possible to adhere said end to a package more than once.

49. A label according to claim 48, wherein said further adhesive medium is a weak adhesive so as to make it possible to detach the label from the package and attach it thereto again without causing any damage thereto.

50. A label according to claim 45, 46, 47, 48 or 49, wherein the label comprises fields in different colours, on which respective prescription data can be printed.

51. A roll comprising a number of labels according to claim 45, 46, 47, 48 or 50.
